# EUROPEAN PATENT SPECIFICATION

(11) **EP 3 046 569 B1**
(45) Date of publication and mention of the grant of the patent: **04.11.2020**
(21) Application number: 14762072.8
(22) Date of filing: 04.09.2014
(51) Int. Cl.: A61K 38/17, A61K 45/06, A61K 31/485, A61P 25/04

(54) **SYNERGISTIC COMBINATION OF ANALGESIC DRUGS**
SYNERGISTISCHE KOMBINATION VON ANALGETIKA
COMBINAISON SYNERGIQUE DE MÉDICAMENTS ANALGÉSIQUES

(30) Priority: 16.09.2013 GB 201316468; 06.11.2013 GB 201319595; 23.05.2014 GB 201409272
(43) Date of publication of application: 27.07.2016
(73) Proprietor: UCL BUSINESS LTD, London W1T 4TP (GB)
(72) Inventor: WOOD, John Nicholas, London NW1 8LS (GB)
(74) Representative: J A Kemp LLP
(86) International application number: PCT/GB2014/052674
(87) International publication number: WO 2015/036734

(56) References cited:
- WO-A1-2012/095781
- WO-A2-2012/004714
- WO-A2-2014/159595
- BERNARD P. ROQUES ET AL: "Inhibiting the breakdown of endogenous opioids and cannabinoids to alleviate pain", NATURE REVIEWS DRUG DISCOVERY, vol. 11, no. 4, 1 April 2012 (2012-04-01), pages 292-310, XP055071274, ISSN: 1474-1776, DOI: 10.1038/nrd3673
- "Poster 45:Phlotoxin 1, a toxin from tarantula venom, is a potent modulator of Nav1.7 sodium channels and a potential analgesic", 15th World Congress on Animal, Plant and Microbial Toxins, Conference , 23 July 2006 (2006-07-23), 28 July 2006 (2006-07-28), pages 220-221, XP002732534, Glasgow, Scotland, United Kingdom Retrieved from the Internet: URL:http://www.angelfish.co.uk/IST/pdf/Fin alabstractbook28thoctforweb.pdf [retrieved on 2014-11-18]
- Howard Smith: "Variations in Opioid Responsivenss", Pain Physician, vol. 11, 1 January 2008 (2008-01-01), pages 237-248, XP055547513,
- Donald D. Price ET AL: "A Comprehensive Review of the Placebo Effect: Recent Advances and Current Thought", ANNUAL REVIEW OF PSYCHOLOGY, vol. 59, no. 1, 1 January 2008 (2008-01-01), pages 565-590, XP055547533, US ISSN: 0066-4308, DOI: 10.1146/annurev.psych.59.113006.095941

## Description

### FIELD OF THE INVENTION

The present invention relates to the treatment and/or prevention of pain.

### BACKGROUND TO THE INVENTION

Electrical signalling in the nervous system relies on the activity of sodium-selective ion channels in neuronal membranes. The voltage-gated sodium channel (VGSC) gene family comprises 9 homologous members *SCN1A-SCN11A*, which encode the sodium selective ion channels Naᵥ1.1-Naᵥ1.9. It is well known that sodium channel blockers are analgesics, but broad spectrum sodium channel blockers such as lidocaine have significant side-effects that often preclude their use.

Thus, cardiotoxicity may result from lidocaine concentrations that are 7-fold higher than those that cause analgesia. Furthermore, lidocaine has been shown to exert analgesic effects *in vivo* that cannot be explained solely by its action on voltage-gated sodium channels (Werdehausen et al, 2012). The activity of broad spectrum sodium channel blockers against targets other than voltage-gated sodium channels may also lead to undesirable side effects.

Other analgesic drug classes such as opioid analgesic drugs are also limited by side-effects, which include constipation, addiction and respiratory depression. Because of these problems, analgesic drug development targeting sodium channels has focused on the sodium channel Naᵥ1.7 encoded by the gene *SCN9A,* as humans who have lost this channel are normal, yet pain-free (Eijkelkamp et al. 2012). Recent studies have also identified pain-free humans with mutations in Naᵥ1.9, whilst Naᵥ1.8 also plays an important role in pain pathways (Leipold et al. 2013).

Naᵥ1.7 is expressed in peripheral sensory neurons innervating the skin, viscera and orofacial region (dorsal root and trigeminal ganglia) as well as sympathetic neurons and olfactory epithelia. A number of human heritable pain disorders map to mutations in *SCN9A,* the gene encoding Naᵥ1.7. Dominant gain-of-function mutations lead to inherited primary erythromelalgia that is characterised by symmetrical burning pain of the feet/lower legs and hands, elevated skin temperature of affected areas, and reddened extremities (Yang et al. 2004). Additionally, other dominant gain-of-function mutations can cause paroxysmal extreme pain disorder that is characterized by episodic burning pain of the rectum, ocular and mandibular regions (Fertleman et al. 2006). Rare recessive loss-of-function mutations cause an inability to experience pain and anosmia.

In transgenic mouse studies, selective knockout of Naᵥ1.7 expression in Naᵥ1.8-positive nociceptors leads to a loss of acute noxious mechanosensation and inflammatory pain, while deletion of Naᵥ1.7 in all sensory neurons leads to an additional loss of noxious thermosensation. Loss of Naᵥ1.7 in sensory and sympathetic neurons leads to an additional loss of neuropathic pain, so that deletion of Naᵥ1.7 in the peripheral neurons of mice mimics the pain free state found in man. In addition, deletion of the gene *Scn9a* in adult mice also leads to a pain-free state, suggesting that Naᵥ1.7 function in adults has an essential role in pain perception (Minett el al, 2012).

Overall, the role of Naᵥ1.7 in human and animal pain perception highlights the potential of Naᵥ1.7 as an important drug target for the treatment of pain with minimal side-effects. However, clinical investigations to date with compounds that inhibit Naᵥ1.7 have indicated that Naᵥ1.7 inhibitors do not produce the complete analgesia associated with loss of Naᵥ1.7 function in human null mutants.

### SUMMARY OF THE INVENTION

Humans without Naᵥ1.7 are pain-free (Cox et al. 2006), but Naᵥ1.7 antagonists have not been found to create equivalently profound analgesia. We reasoned that other roles for *SCN9A* and Naᵥ1.7 may contribute to the analgesia found in human *SCN9A* loss-of-function mutants. The present inventor investigated the role of Naᵥ1.7 in the perception of pain and discovered that *Scn9a* functional gene ablation in mice, with a loss of Naᵥ1.7 channel activity has profound effects on transcription in sensory neurons involved in pain responses.

In particular, preproenkephalin (*Penk1*) mRNA expression in murine sensory neurons is massively increased in mice lacking the sodium channel Naᵥ1.7. This effect was specifically linked to the deletion of *Scn9a* encoding Naᵥ1.7, because the deletion of *Scn10a* encoding Naᵥ1.8 or *Scn11a* encoding Naᵥ1.9 sodium channels in mice did not lead to any significant change in preproenkephalin mRNA levels. The levels of Met-enkephalin peptide present in sensory neurons were investigated using immunocytochemistry. A dramatic increase in immunoreactive Met-enkephalin was detected in dorsal root ganglion neurons and their central terminals in Naᵥ1.7 null mutant mice compared to wild type littermates using specific antisera directed against Met-enkephalin. Leu- and Met-enkephalins are the metabolic products of preproenkephalin that bind to opioid receptors to suppress pain. Opioid receptors are G-protein coupled receptors that act through a number of pathways to suppress pain perception. Following this discovery, it was further found that the administration of naloxone, an opioid receptor antagonist that has no effects on normal pain perception at the dose used, substantially reverses the pain-free state in mice lacking Naᵥ1.7, suggesting that opioid-based analgesia caused by high levels of endogenous enkephalins contributes to the pain-free state experienced by human loss-of-function Naᵥ1.7 mutants. In order to examine this potential mechanism in humans, a person with loss-of-function Naᵥ1.7 mutations who is pain-free was also treated intravenously with naloxone. This resulted in a dramatic sensitisation of pain thresholds, supporting the case that enkephalins contribute substantially to the pain-free state found in human Naᵥ1.7 null mutants. Control normal humans were unaffected in their pain behaviour by treatment with similar levels of naloxone. Importantly, because the pain-free state has been maintained over many years, this suggests that mimicking the endogenous mechanisms that contribute to Naᵥ1.7-dependent CIP (congenital insensitivity to pain) will provide a form of analgesia that does not have associated side-effect or tolerance problems.

In view of these novel and surprising findings, the present inventor treated wild-type mice with a combination of a Naᵥ1.7 inhibitor (Phlotoxin-1) which shows sub-micromolar selective affinity for Nav1.7 compared to other expressed sodium channels (Escoubas et al. 2006, Bosmans et al. 2005), and an opioid analgesic drug in low doses (which when administered alone provided no or little detectable analgesic effect). Within minutes of administration, a profound analgesic effect was observed in the mice tested with the Hargreaves apparatus that measures acute thermal pain (Minett et al. 2011). These results demonstrate that a combination of a Naᵥ1.7 inhibitor and an opioid analgesic drug provides an enhanced and effective therapeutic approach to the treatment and/or prevention of pain, due to the synergistic interaction between the drugs. It also demonstrates that low dose opioid analgesic drugs that have few or no undesirable side effects can be potentiated in their analgesic activity by co-administration with a selective sodium channel blocker that inhibits Naᵥ1.7.

Enkephalins have a short *in vivo* half-life due to degradation by enzymes such as aminopeptidase N (APN), neutral endopeptidase (NEP), dipeptidyl peptidase 3 (DPP3), carboxypeptidase A6 (CPA6), and angiotensin-converting enzyme (ACE). Collectively, these enzymes are known as the enkephalinases. It is possible to enhance enkephalin stability by blocking the activity of the enkephalinases with enkephalinase inhibitors (Roques et al. 2012). Administration of an enkephalinase inhibitor to a patient will lead to a rise in enkephalin levels, as occurs when an opioid analgesic drug is administered.

The present inventors therefore also treated wild-type mice with a combination of a Naᵥ1.7 inhibitor and thiorphan, an enkephalinase inhibitor. A profound analgesic effect was observed in the mice tested using the Hargreaves apparatus with this combination of drugs. These results demonstrate that a combination of a Naᵥ1.7 inhibitor and an enkephalinase inhibitor also provides an enhanced and effective therapeutic approach to the treatment and/or prevention of pain, due to the synergistic interaction between the drugs.

The present inventors then explored a further range of pain models using Naᵥ1.7 antagonists in combination with opioids or enkephalinase inhibitors. These tests included models of acute pain (Hargreaves test and formalin test - Minett et al. 2012)), models of inflammatory pain (complete Freunds adjuvant [CFA] test and formalin test) and models of neuropathic pain (spinal nerve transection at the fifth lumbar segment). In addition, models of osteoarthritis (monoiodoacetate evoked-osteoarthritis) pain were explored. In all cases, the combination of a Naᵥ1.7 antagonist and an opioid or enkephalinase inhibitor led to a reversal of pain. In addition, the inventors examined the effect of intrathecal administration of Phlotoxin-1 and buprenorphine alone or in combination, and also observed a synergistic effect of applying the Naᵥ1.7 antagonist with an opioid on pain behaviour elicited by a CFA model of inflammatory pain. The drug effects reduced pain behaviour for 4 - 6 hours when applied locally to the site of CFA injection.

Accordingly, the present invention provides a pharmaceutical composition comprising (a) an enkephalinase inhibitor, and (b) a selective Naᵥ1.7 inhibitor.

The invention further provides pharmaceutical composition comprising (a) an enkephalinase inhibitor, and (b) a selective Naᵥ1.7 inhibitor, for use in treating pain.

The invention further provides an enkephalinase inhibitor for use in treating pain in combination with a selective Naᵥ1.7 inhibitor.

The invention further provides a selective Naᵥ1.7 inhibitor, for use in treating pain in combination with an enkephalinase inhibitor.

### BRIEF DESCRIPTION OF THE FIGURES

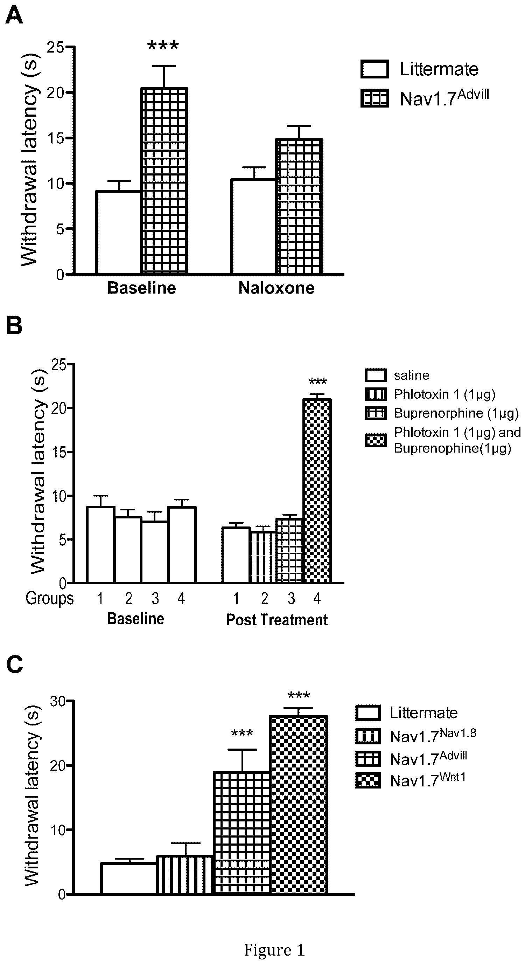

Figure 1 shows the results from Example 2 and that opioid analgesia contributes to the pain-free state found in *Scn9a* null mutant mice, and a sodium channel inhibitor that blocks Naᵥ1.7 and an opioid analgesic drug act synergistically to cause profound analgesia in a murine acute thermal pain model.
Figure 1A shows that injection of 2mg/kg naloxone, an opioid antagonist partially reverses the analgesia (p=0.009) experienced by Naᵥ1.7 tissue-specific null mutant mice (Naᵥ1.7^{Advill} mice) in a murine acute thermal pain model but does not alter pain thresholds in normal mice. "Baseline" denotes the results of Hargreaves testing before naloxone treatment. "Naloxone" denotes the results of Hargreaves testing after naloxone treatment. Results are presented as mean ± SEM. *** p < 0.001.
Figure 1B shows that low doses of both the Naᵥ1.7 antagonist Phlotoxin-1 (protein sequence ACLGQWDSCDPKASKCCPNYACEWKYPWCRYKLF - SEQ ID No.1) and the opioid analgesic drug buprenorphine caused profound analgesia in a murine acute thermal pain model when administered intraplantarly together, but not when administered singly. "Baseline" denotes the results of Hargreaves testing before administered of stated treatment. "Post Treatment" denotes the results of Hargreaves testing after administered of stated treatment. Results are presented as mean ± SEM. *** p < 0.001.
Figure 1C shows that conditional gene deletion of Naᵥ1.7 (Naᵥ1.7^{Advill} and Naᵥ1.7^{Wnt1} mice) results in the same analgesic effect in a murine acute thermal pain model as co-administration of Phlotoxin-1 1 and buprenorphine. Data were analysed by two-way analysis of variance followed by Bonferroni post-hoc test. Results are presented as mean ± SEM. *** p < 0.001.

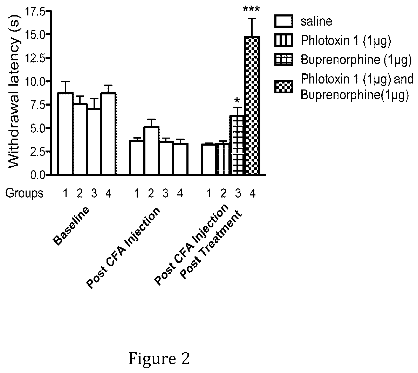

Figure 2 shows further results from Example 2 and that inflammatory hyperalgesia in a murine thermal pain model is also reversed by low dose intraplantar (1 microgram, 0.25 nMols) Phlotoxin-1 1 and buprenorphine (1 microgram, 2nMols), but single agents have much lesser effects, in a murine acute thermal pain model. "Baseline" denotes the results of Hargreaves testing before administration of the stated treatment. "Post CFA Injection" denotes the results of Hargreaves testing after administration of CFA. "Post Treatment" denotes the results of Hargreaves testing after administration of the stated treatment to the animal that received intraplantar CFA injections. CFA, Complete Freund's Adjuvant. Data were analysed by two-way analysis of variance followed by Bonferroni post-hoc test. Results are presented as mean ± SEM. * p < 0.05; *** p < 0.001.

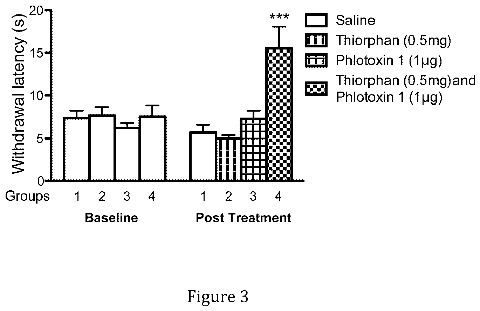

Figure 3 shows results from Example 4 and that the Phlotoxin-1 sodium channel inhibitor that blocks Naᵥ1.7 and an enkephalinase inhibitor, thiorphan (20mg/kg), act synergistically to cause profound analgesia in a murine acute thermal pain model. "Baseline" denotes the results of Hargreaves testing before administration of the stated treatment. "Post Treatment" denotes the results of Hargreaves testing after administration of the stated treatment. Data were analysed by two-way analysis of variance followed by Bonferroni post-hoc test. Results are presented as mean ± SEM. *** p < 0.001.

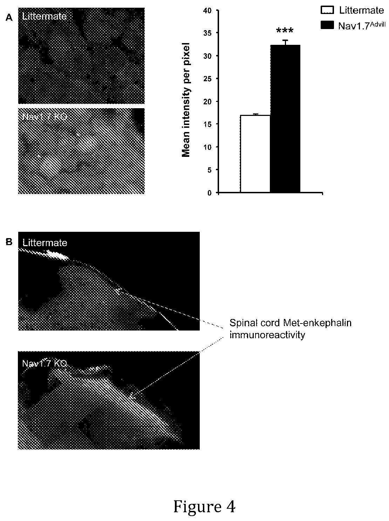

Figure 4 shows the results from Example 7, in which expression of Met-enkephalin peptides was detected in the dorsal root ganglia (Figure 4A) and spinal cord (Figure 4B) of Naᵥ1.7 null mutant and normal mice. Deleting Naᵥ1.7 increases Met-enkephalin immunoreactivity in the DRG and spinal cord of Nav1.7 null mutant mice as compared to normal mice.

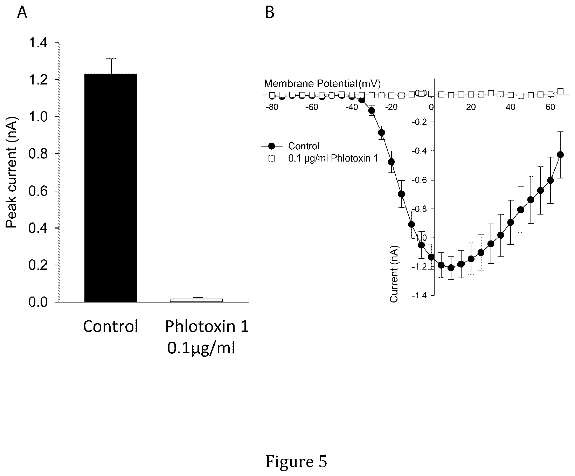

Figure 5 shows the results from Example 8, and demonstrates that Phlotoxin-1 at a concentration of 0.25µM completely inhibits functional Naᵥ1.7 voltage-gated channel activity in a Naᵥ1.7 transfected cell line.

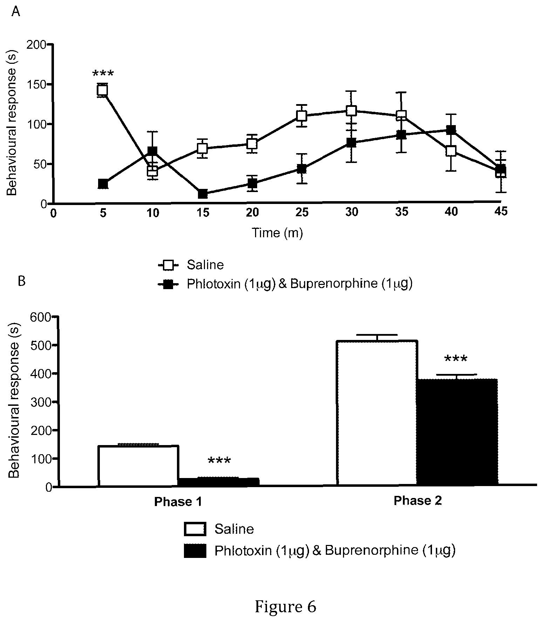

Figure 6 shows the results from Example 9, in which the formalin test was applied to mice. Figure 6A shows that co-administration of Phlotoxin-1 and buprenorphine results in an attenuation and a delay in onset of the pain behaviour associated with the formalin test. Figure 6B shows that overall both the first (0 - 10 minutes post formalin injection) and second (10 - 45 minutes post formalin injection) phases are lowered by co-administration of Phlotoxin-1 and buprenorphine. The first phase is considered to reflect acute pain, whilst the second phase of pain behaviour (paw licking and biting) reflects central modulation of pain pathways. Data were analysed by two-way analysis of variance followed by Bonferroni post-hoc test. Results are presented as mean ± SEM. *** P<0.001.

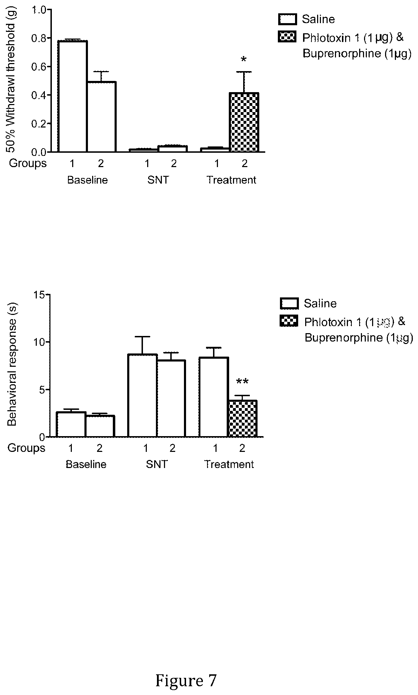

Figure 7 shows the results from tests on neuropathic pain in Example 10. Following sciatic nerve transection, mechanical allodynia (Figure 7A) and cold allodynia (Figure 7B) were reversed by application of buprenorphine and Phlotoxin-1. "Baseline" denotes the results of von Frey or Acetone testing before spinal nerve transaction at the fifth lumber segment. "SNT" denotes the results of von Frey or Acetone testing after spinal nerve transaction at the fifth lumber segment. "Treatment" denotes the results of von Frey or Acetone testing following co-administration of Phlotoxin-1 and buprenorphine to animals with transacted spinal nerves at the fifth lumber segment. Data were analysed by two-way analysis of variance followed by Bonferroni post-hoc test. Results are presented as mean ± SEM. * P<0.05, **P<0.01.

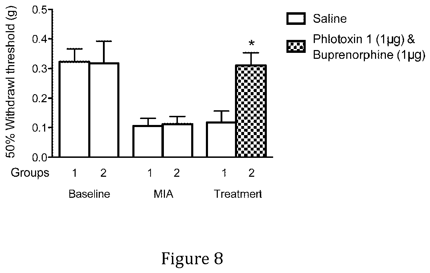

Figure 8 shows the results from Example 11, in which osteoarthritis pain (21 days) was reversed by co-administration Phlotoxin-1 plus buprenorphine. "Baseline" denotes the results of von Frey testing before injection of MIA, "MIA" denotes the results of von Frey testing 21 days after injection of MIA and "MIA post treatment" shows the results of von Frey testing 21 days after injection of MIA following co-administration of Phlotoxin-1 and buprenorphine. Data were analysed by two-way analysis of variance followed by Bonferroni post-hoc test. Results are presented as mean ± SEM. * P<0.05.

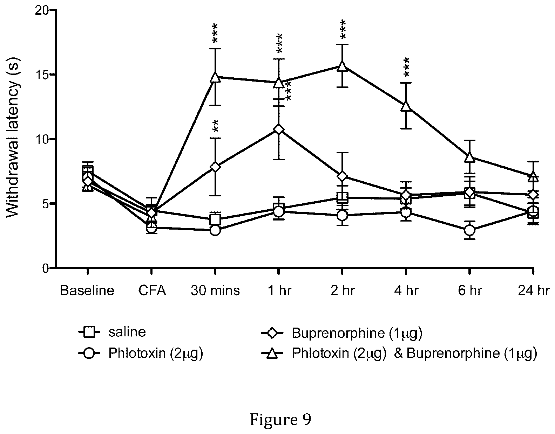

Figure 9 shows the results from Example 12. Complete Freunds Adjuvant (CFA)-evoked pain was synergistically blocked by co-administration buprenorphine and Phlotoxin-1. "Baseline" denotes the results of Hargreaves testing before intraplantar injection of CFA, "CFA" denotes the results of Hargreaves testing after intraplantar injection of CFA and subsequent time points denotes measurement take after intraplantarco-administration of Phlotoxin-1 and buprenorphine to animals injected with CFA. Data were analysed by two-way analysis of variance followed by Bonferroni post-hoc test. Results are presented as mean ± SEM **P<0.01, *** P<0.001.

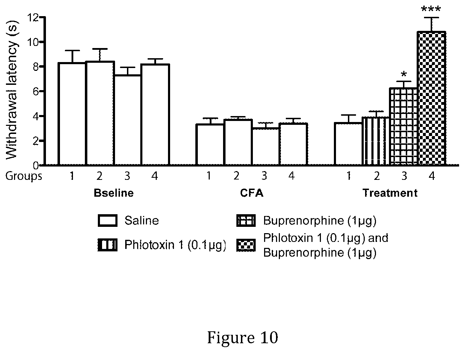

Figure 10 shows the results from Example 13. Complete Freunds Adjuvant (CFA)-evoked pain was synergistically blocked by intrathecal co-administration buprenorphine and Phlotoxin-1. "Baseline" denotes the results of testing before intraplantar injection of CFA, "CFA" denotes the results of testing after intraplantar injection of CFA. "Post CFA injection, Post Treatment" denotes the results of testing after co-administration of Phlotoxin-1 and buprenorphine to animals injected with CFA. Data were analysed by two-way analysis of variance followed by Bonferroni post-hoc test. Results are presented as mean ± SEM **P<0.01, *** P<0.001.

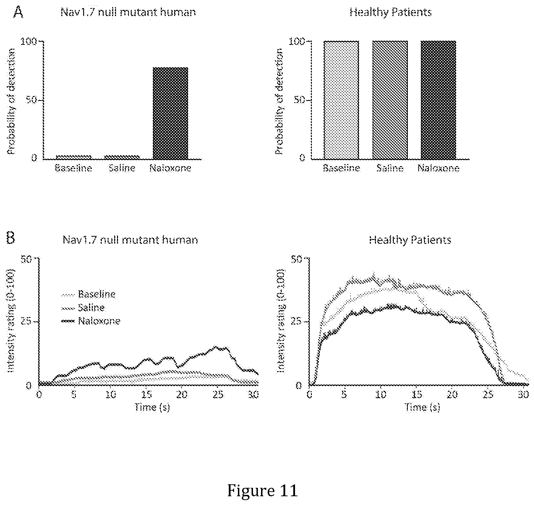

Figure 11 shows the results from Example 14. These human data show that the opioid antagonist naloxone sensitises Naᵥ1.7 null mutant humans to noxious stimuli (phasic radiant heat in Figure 11A, tonic radiant heat in Figure 11B) but has no effect on healthy patients.

### DETAILED DESCRIPTION OF THE INVENTION

The present invention relates to combinations of (a) an enkephalinase inhibitor, and (b) a selective Naᵥ1.7 inhibitor, typically in a pharmaceutical composition, and their use in the treatment of pain.

The invention also relates to combinations of (a) an opioid analgesic drug and an enkephalinase inhibitor, and (b) a selective Naᵥ1.7 inhibitor, typically in a pharmaceutical composition, and their use in the treatment of pain.

### Opioid analgesic drugs

Opioid analgesic drugs are a well-known class of compounds that bind to opioid receptors, and can thus be used to treat pain. Their potent analgesic effects are accompanied by side-effects such as constipation, respiratory depression and addiction, which limit their use for many pain patients. Usually, short acting opioids such as morphine are used to assess clinical dosing needs whilst long-lasting opioids are then administered at appropriate doses.

Examples of opioids analgesic drugs include morphine, dimorphine, fentanyl, tramadol, 2,4-dinitrophenylmorphine, 6-MDDM, chlornaltrexamine, desomorphine, dihydromorphine, hydromorphinol, methyldesorphine, N-phenethylnormorphine, RAM-378, acetylpropionylmorphine, dihydroheroin, dibenzoylmorphine, dipropanoylmorphine, heroin, nicomorphine, codeine, 6-MAC, benzylmorphine, codeine methylbromide, dihydroheterocodeine, ethylmorphine, heterocodeine, pholcodine, myrophine, 14-cinnamoyloxycodeinone, 14-ethoxymetopon, 14-methoxymetopon, PPOM, 7-spiroindanyloxymorphone, acetylmorphone, codeinone, conorphone, codoxime, thebacon, hydrocodone, hydromorphone, metopon, morphinone, N-phenethyl-14-ethoxymetopon, oxycodone, oxymorphone, pentamorphone, semorphone, chloromorphide, 14-hydroxydihydrocodeine, acetyldihydrocodeine, dihydrocodeine, nalbuphine, nicocodeine, nicodicodeine, oxymorphazone, 1-iodomorphine, M6G, 6-MAM, norcodeine, normorphine, morphine-N-oxide, cyclorphan, DXA, levorphanol, levophenacylmorphan, levomethorphan, norlevorphanol, oxilorphan, phenomorphan, furethylnorlevorphanol, xorphanol, butorphanol, cyprodime, drotebanol, 7-PET, acetorphine, BU-48, buprenorphine, cyprenorphine, dihydroetorphine, etorphine, norbuprenorphine.

Preferably the opioid analgesic drug is morphine, diamorphine, oxycodone, fentanyl, tramadol or buprenorphine.

Alternatively, the opioid analgesic drug may be an enkephalin analogue, such as the compound with the following structure: 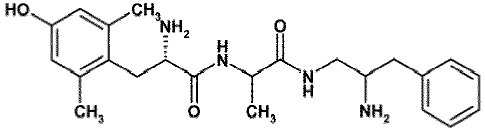 or a tautomer, an ionic form, or a pharmaceutically acceptable salts thereof, as described in WO 2013/008123.

### Enkephalinase inhibitors

Enkephalins are endogenous analgesic peptides acting at delta and mu opioid receptors. They have the structure Tyr-Gly-Gly-Phe-Met (Met-enkephalin) or Tyr-Gly-Gly-Phe-Leu (Leu-enkephalin). Enkephalins are digested *in vivo* by protease enzymes known as the enkephalinase class of enzymes, which includes aminopeptidase N (APN), neutral endopeptidase (NEP), dipeptidyl peptidase 3 (DPP3), carboxypeptidase A6 (CPA6) and angiotensin-converting enzyme (ACE). Compounds that inhibit the activity of one or more enkephalinase enzymes are known as enkephalinase inhibitors.

Typically, an enkephalinase inhibitor is a compound with an IC₅₀ of less than 1µM on enkephalinase enzymes.

The activity of a particular compound as an enkephalinase inhibitor can be determined using techniques known to those skilled in the art, for example the methods described below in Example 5.

Examples of enkephalinase inhibitors include ubenimex (also known as Bestatin), BL-2401, kelatorphan, D-phenylalanine, racecadotril, RB-101, RB-120, RB-3007, thiorphan, tynorphin, opiorphin, spinorphin and NH₂-CH-Ph-P(O)(OH)CH₂-CHCH₂Ph( p-Ph)-CONH-CH-CH₃-COOH (P8B). Further examples of enkephalinase inhibitors can be found in EP 0 161 769 A1.

Preferably, the enkephalinase inhibitor is thiorphan, which has the following structure:

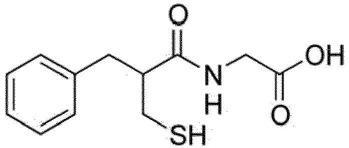

Two further examples of enkephalinase inhibitors are the compounds N-(3-mercapto-5-methyl-oxohexyl)-L-phenyl alanine (C20) and N-(3-mercapto-3-trifluoromethyl-1-oxopropyl)-L-tyrosine (NESS002ie) which are depicted below (Tambaro et al. 2013).

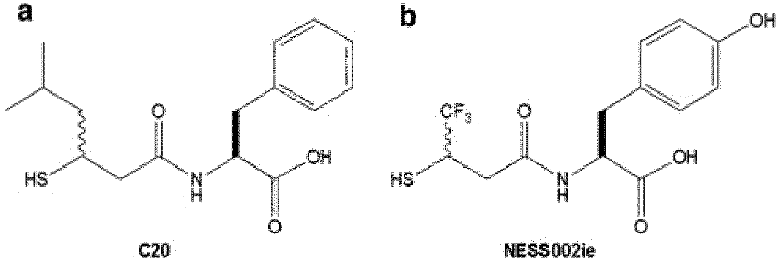

### Selective Naᵥ1.7 inhibitors

A selective Naᵥ1.7 inhibitor is a compound that can block Naᵥ1.7 sodium-selective ion channels without blocking other voltage-gated sodium channels, such as Naᵥ1.1, Naᵥ1.3, Naᵥ1.4, Naᵥ1.5 or Naᵥ1.6.

Selective Naᵥ1.7 inhibitors typically do not have local anaesthetic activity, in contrast to non-selective sodium channel blockers such as lidocaine. Although, local anaesthetics are a very effective way to treat pain, they block all sensation and are thus impractical for most types of pain. Furthermore, the undesirable side effects, particularly cardiac side effects, observed with non-selective sodium channel blockers such as lidocaine are not observed with selective Naᵥ1.7 inhibitors.

Typically, a selective Naᵥ1.7 inhibitor is a compound with an IC₅₀ of less than 100nM on expressed human Naᵥ1.7 channels

Typically, a selective Naᵥ1.7 inhibitor has a selectivity for Naᵥ1.7 over other sodium channels of greater than 5 fold, preferably greater than 10 fold, more preferably greater than 20 fold, greater than 50 fold, greater than 100 fold, greater than 200 fold, greater than 500 fold, greater than 750 fold or greater than 1000 fold. In particular, the selective Naᵥ1.7 inhibitor may have greater than 5 fold, preferably greater than 10 fold, more preferably greater than 20 fold, greater than 50 fold, greater than 100 fold, greater than 200 fold, greater than 500 fold, greater than 750 fold or greater than 1000 fold selectivity for Naᵥ1.7 over Naᵥ1.1, Naᵥ1.3 Naᵥ1.4, Naᵥ1.5 or Naᵥ1.6 or over all of Naᵥ1.1, Naᵥ1.3, Naᵥ1.4, Naᵥ1.5 and Naᵥ1.6. In the case of a selective Naᵥ1.7 inhibitor that is an antibody, selectivity will typically be greater than 500 fold, greater than 750 fold or greater than 1000 fold for Naᵥ1.7 over Naᵥ1.1, Naᵥ1.3, Naᵥ1.4, Naᵥ1.5 or Naᵥ1.6 or over all of Naᵥ1.1, Naᵥ1.3, Naᵥ1.4, Naᵥ1.5 and Naᵥ1.6.

The activity and/or selectivity of a particular compound as a Naᵥ1.7 inhibitor can be determined using techniques known to those skilled in the art, for example the methods described below in Example 3.

Examples of selective Naᵥ1.7 inhibitors include the compounds described in the following documents : WO 2012/004714; WO 2007/109324; WO 2012/007868; WO 2012/007869; WO 2012/004706; WO 2012/007877; WO 2010/137351; WO 2009/005460; WO 2013/102826; WO 2013/134518; WO 2012/004664; WO 2010/079443; WO 2009/145721; WO 2007/109324; WO 2013/093688; WO 2012/095781; WO 2012/039657; WO 2009/000038; WO 2012/035023; and Bioorg Med Chem Lett. 2013 Jun 15;23(12):3640-5. doi: 10.1016/j.bmcl.2013.03.121. Epub 2013 Apr 5.

Thus, the selective Naᵥ1.7 inhibitor can for example be a compound of formula (I) as described in WO 2012/004714: 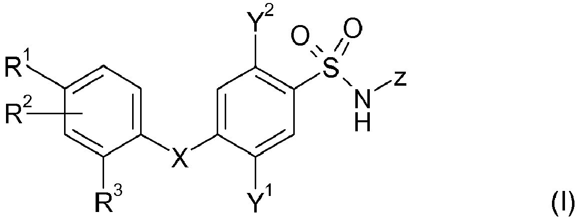 or a pharmaceutically acceptable salt thereof, wherein:
Z is a 'C-linked' 5- or 6-membered heteroaryl comprising (a) one or two nitrogen atoms or, when 5-membered, (b) one or two nitrogen atoms and one sulphur atom, said heteroaryl being optionally substituted on a ring carbon atom by F or CI;
Y¹ is CN, F, CI or R⁴ ;
Y² is H or F;
X is CH₂ or S;
R¹ and R² are independently H, CI, F, R⁵, Ar or Het¹;
R³ is H, F, R⁵, Ar or Het¹;
R⁴ is (Ci-C₄)alkyl optionally substituted by one to three F;
R⁵ is (Ci-C₄)alkyl, optionally substituted by one to three F; or (Ci-C₄)alkyloxy, optionally substituted by one to three F;
Ar is phenyl optionally substituted by one to three atoms or groups selected from CI, F or R⁵;
Het¹ is a 'C-linked' 5- or 6-membered heteroaryl group comprising one or two nitrogen atoms, being optionally substituted by one to three substituents selected from A or B; A is attached to a Het¹ ring carbon and is selected from Het², NH₂ and R⁴;
B is attached to a Het¹ ring nitrogen and is selected from 'C-linked' Het² and R⁴; and
Het² is a 'C-linked' 3- to 8-membered saturated heterocyclic group comprising one or two ring nitrogen atoms, or (b) one oxygen atom and one or two nitrogen atoms, said heterocyclic group being optionally substituted by R⁴.

Another preferred selective Naᵥ1.7 inhibitor is the peptide Phlotoxin-1, which has the sequence ACLGQWDSCDPKASKCCPNYACEWKYPWCRYKLF ("Phlotoxin-1, a toxin from tarantula venom, is a potent modulator of Nav1.7 sodium channels and a potential analgesic", Escoubas P et al., poster presentation at 15th World Congress on Animal, Plant and Microbial Toxins, Glasgow, Scotland, 23-28 July 2006).

A further preferred selective Naᵥ1.7 inhibitor is µ-SLPTX-Ssm6a, a 46-residue peptide from centipede venom that inhibits Naᵥ1.7 with an IC₅₀ of ∼25 nM. (Yang et al. 2013).

A further preferred type of selective Naᵥ1.7 inhibitor is an antibody that specifically binds Naᵥ1.7 and is selective for Naᵥ1.7 over other sodium channels (see above), including human monoclonal antibodies that target the Nav1.7 voltage sensor S3-S4 loop (Lee et al. 2014).

The term "antibody" as referred to herein includes whole antibodies and any antigen binding fragment (*i*.*e.*, "antigen-binding portion") or single chains thereof. An antibody refers to a glycoprotein comprising at least two heavy (H) chains and two light (L) chains inter-connected by disulfide bonds, or an antigen-binding portion thereof. Each heavy chain is comprised of a heavy chain variable region (abbreviated herein as VH) and a heavy chain constant region. Each light chain comprises a light chain variable region (abbreviated herein as VL) and a light chain constant region. The variable regions of the heavy and light chains contain a binding domain that interacts with an antigen. The VH and VL regions can be further subdivided into regions of hypervariability, termed complementarity determining regions (CDR), interspersed with regions that are more conserved, termed framework regions (FR). The constant regions of the antibodies may mediate the binding of the immunoglobulin to host tissues or factors, including various cells of the immune system (e.g., effector cells) and the first component (Clq) of the classical complement system.

An antibody of the invention may be a monoclonal antibody or a polyclonal antibody. Preferably, an antibody of the invention is a monoclonal antibody. Polyclonal antibodies are antibodies that are derived from different B cell lines. A polyclonal antibody may comprise a mixture of different immunoglobulin molecules that are directed against a specific antigen. The polyclonal antibody may comprise a mixture of different immunoglobulin molecules that bind to one or more different epitopes within an antigen molecule. Polyclonal antibodies may be produced by routine methods such as immunisation with the antigen of interest. For example a mouse or other animal capable of expressing human antibody sequences may be immunised with Naᵥ1.7 or epitope-containing portions thereof. Blood may be subsequently removed and the Ig fraction purified.

Monoclonal antibodies are immunoglobulin molecules that are identical to each other and have a single binding specificity and affinity for a particular epitope. Monoclonal antibodies (mAbs) of the present invention can be produced by a variety of techniques, including conventional monoclonal antibody methodology.

The term "antigen-binding portion" of an antibody refers to one or more fragments of an antibody that retain the ability to specifically bind to an antigen, such as CD40. It has been shown that the antigen-binding function of an antibody can be performed by fragments of a full-length antibody. Examples of binding fragments encompassed within the term "antigen-binding portion" of an antibody include a Fab fragment, a F(ab')₂ fragment, a Fab' fragment, a Fd fragment, a Fv fragment, a dAb fragment and an isolated complementarity determining region (CDR). Single chain antibodies such as scFv and heavy chain antibodies such as VHH and camel antibodies are also intended to be encompassed within the term "antigen-binding portion" of an antibody. These antibody fragments may be obtained using conventional techniques known to those of skill in the art, and the fragments may be screened for utility in the same manner as intact antibodies.

An antibody of the invention may be prepared, expressed, created or isolated by recombinant means, such as (a) antibodies isolated from an animal (e.g., a mouse) that is transgenic or transchromosomal for the immunoglobulin genes of interest or a hybridoma prepared therefrom, (b) antibodies isolated from a host cell transformed to express the antibody of interest, e.g., from a transfectoma, (c) antibodies isolated from a recombinant, combinatorial antibody library, and (d) antibodies prepared, expressed, created or isolated by any other means that involve splicing of immunoglobulin gene sequences to other DNA sequences.

An antibody of the invention may be a chimeric antibody, a CDR-grafted antibody, a nanobody, a human or humanised antibody or an antigen-binding portion of any thereof.

In one preferred embodiment, the antibody of the invention is a human antibody. The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences.

Such a human antibody may be a human monoclonal antibody. Such a human monoclonal antibody may be produced by a hybridoma, which includes a B cell obtained from a transgenic nonhuman animal, e.g., a transgenic mouse, having a genome comprising a human heavy chain transgene and a light chain transgene fused to an immortalized cell.

Human antibodies may be prepared by *in vitro* immunisation of human lymphocytes followed by transformation of the lymphocytes with Epstein-Barr virus.

The term "human antibody derivatives" refers to any modified form of the human antibody, e.g., a conjugate of the antibody and another agent or antibody.

An antibody of the invention can also be a "humanised" antibody. To obtain a humanised antibody, an animal is immunised and a monoclonal antibody obtained. This will be not be a human antibody but rather an antibody of the immunised species, e.g., a mouse antibody. Recombinant antibody engineering techniques known in the art are then used to obtain an antibody with the same specificity in a more human background, e.g., by grafting the CDRs from the original antibody into human framework regions and/or by combining with human constant regions.

Binding affinity may be quantified by determining the dissociation constant (Kd) for an antibody and its target. Similarly, the specificity of binding of an antibody to its target may be defined in terms of the comparative dissociation constants (Kd) of the antibody for its target as compared to the dissociation constant with respect to the antibody and another, non-target molecules.

Specific binding may be assessed with reference to binding of the antibody to a molecule that is not the target. This comparison may be made by comparing the ability of an antibody to bind to the target and to another molecule. This comparison may be made as described above in an assessment of Kd or Ki. The other molecule used in such a comparison may be any molecule that is not the target molecule. In particular, these techniques can be used to assess selectivity for Naᵥ1.7 over other sodium channels.

Antibodies of the invention will desirably bind to Naᵥ1.7 with high affinity, preferably in the nanomolar or picomolar range, e.g., with an affinity constant (K_{D}) of 100nM or less, 10nM or less, 1nM or less, 500pM or less or 100pM or less, measured by surface plasmon resonance or any other suitable technique.

Antibodies of the invention can be tested for binding to peptides derived from Naᵥ1.7 by, for example, standard ELISA or Western blotting. An ELISA assay can also be used to screen for hybridomas that show positive reactivity with a peptide derived from the target protein. Either full-length Naᵥ1.7 or any suitable peptide fragment of Naᵥ1.7 may be used. For example, the peptide may comprise , consist of, or consist essentially of, the voltage sensor paddle region (S3-S4 loop) of sequence LSLVELFLADVEGLSVLR (SEQ ID No.2) against which function blocking selective monoclonal antibodies can be produced (Lee et al. 2014) . The binding specificity of an antibody may also be determined by monitoring binding of the antibody to cells expressing the target protein, for example by flow cytometry. Following these screens, function blocking activity can be assessed electrophysiologically with expressed human Naᵥ1.7 channels, and behavioral assays as described (Minett et al., 2011) can be used to test the antibody alone and in combination with low dose opioid drugs and/or enkephalinase inhibitors.

Once a suitable antibody has been identified and selected, the amino acid sequence of the antibody may be identified by methods known in the art. The genes encoding the antibody can be cloned using degenerate primers. The antibody may be recombinantly produced by routine methods, for example by expression in a cell line such as a Chinese Hamster Ovary (CHO) cell line.

Examples of suitable antibodies are those described in CN 102781963 A and in Lee et al, 2014.

As long as they act as selective inhibitors of Naᵥ1.7, antibodies of the invention may target any part of Naᵥ1.7, i.e. their epitope may be anywhere on the Naᵥ1.7 protein that enables the antibody to bind in a way that blocks Naᵥ1.7 function as defined herein. For example, the antibodies may bind the sensor paddle region (S3-S4 loop) (LSLVELFLADVEGLSVLR - SEQ ID No.2) against which it is known that function blocking selective monoclonal antibodies can be produced (Lee et al. 2014).

### Therapeutic indications

The present invention is directed towards the treatment and/or prevention of pain in a subject. Typically, the subject is a human or non-human animal. The term "non-human animal" includes all vertebrates, e.g., mammals and non-mammals, including non-human primates such as monkeys and apes, sheep, dogs, cats, horses, cows, pigs, chickens, amphibians, reptiles, etc. Administration to humans is preferred.

Many different types of pain can be treated or prevented, as can pain originating from many different sources. Treatment and/or prevention of pain conditions can be achieved by administration of combinations of (a) an enkephalinase inhibitor and optionally an opioid analgesic drug, and (b) a selective Naᵥ1.7 inhibitor, according to the invention.

Typically, the pain is acute pain, inflammatory pain or neuropathic pain. Preferably, the pain is acute pain inflammatory pain. Alternatively, the pain may be neuropathic pain. Acute pain can be, for example, post-operative pain. Inflammatory pain is typically caused by osteoarthritis, rheumatoid arthritis, Crohn's disease or fibromyalgia. Neuropathic pain is typically caused by diabetic neuropathy, trigeminal neuralgia, HIV-evoked neuropathy or antiviral neuropathy. Alternatively, neuropathic pain may be caused by nerve trauma.

It is believed that the enkephalinase inhibitor, opioid analgesic drug and selective Naᵥ1.7 inhibitor of the invention act principally on peripheral sensory and sympathetic neurons, thereby treating pain originating at those sites. Typically, therefore, the pain treated according to the present invention is pain originating at peripheral sensory and/or sympathetic neurons that occurs with acute, inflammatory or neuropathic noxious stimuli.

Further, due to the fact that the opioid analgesic drug can be used at low or sub-clinical doses, the combination comprising the opioid analgesic drug is particularly suitable for use in patients who are intolerant to opioid analgesic drugs. Such patients constitute around 20% of the population.

A combination of (a) an enkephalinase inhibitor and optional opioid analgesic drug, and (b) a selective Naᵥ1.7 inhibitor according to the invention may also be administered prophylactically, to prevent pain that does not yet exist or is at a low level but is expected to arise or worsen, e.g., as a result of a surgical procedure or the worsening of a medical condition.

Treatment or prevention of pain is understood to be effective if pain is either reduced or eliminated. Reduction or elimination of pain can be measured by any suitable technique known in the art, for example via the techniques known as the McGill pain questionnaire or McGill pain index. The McGill Questionnaire consists primarily of three major classes of world descriptors-sensory, affective and evaluative, which are used by patients to specify pain experience. The three major measures are the pain rating index, the number of words chosen and the present pain intensity based on a 1-5 intensity scale.

### Pharmaceutical compositions

The enkephalinase inhibitor, optional opioid analgesic drug and selective Naᵥ1.7 inhibitor according to the invention may be administered by any suitable route, depending on the nature of the disorder to be treated. For example, administration may be oral (as syrups, tablets, capsules, lozenges, controlled-release preparations, fast-dissolving preparations, etc), topical (as creams, ointments, lotions, nasal sprays or aerosols, etc.), by injection (subcutaneous, intradermal, intramuscular, intravenous, etc.), via transdermal patch or by inhalation (as a dry powder, a solution, a dispersion, etc.).

The enkephalinase inhibitor, optional opioid analgesic drug and selective Naᵥ1.7 inhibitor may be administered together in the same pharmaceutical composition or in different compositions intended for simultaneous, separate, or sequential administration, by the same or a different route. For example, one drug may be administered systemically whilst the other is administered locally. Preferably, the enkephalinase inhibitor, optional opioid analgesic drug and selective Naᵥ1.7 inhibitor are administered at the same time.

The enkephalinase inhibitor, optional opioid analgesic drug and selective Naᵥ1.7 inhibitor are typically presented in unit dosage form, for convenience.

Pharmaceutical compositions comprising (a) the enkephalinase inhibitor and optional opioid analgesic drug, and (b) the selective Naᵥ1.7 inhibitor may be prepared by any suitable method known to those of skill in the art. The pharmaceutical compositions typically comprise one or more pharmaceutically acceptable carriers. Preferably, each carrier is suitable for administration orally or by injection or through transdermal patches , e.g. subcutaneous, intradermal, intramuscular or intravenous administration.

Preferred pharmaceutically acceptable carriers comprise aqueous carriers or diluents. Examples of suitable aqueous carriers that may be employed in the pharmaceutical compositions of the invention include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. In many cases, it will be preferable to include isotonic agents, for example, sugars, polyalcohols such as mannitol, sorbitol, or sodium chloride in the composition.

Pharmaceutical compositions typically must be sterile and stable under the conditions of manufacture and storage. The composition can be formulated as a solution, microemulsion, liposome, or other ordered structure suitable to high drug concentration.

Pharmaceutical compositions of the invention may comprise additional active ingredients, such as an additional therapeutic or prophylactic agent intended, for example, for the treatment of the same condition or a different one, or for other purposes such as amelioration of side effects.

### Dosages and dosage regimes

Suitable dosages of enkephalinase inhibitor, opioid analgesic drug and selective Naᵥ1.7 inhibitor may be determined by a skilled medical practitioner.

Actual dosage levels of the active ingredients in the pharmaceutical compositions of the present invention may be varied so as to obtain an amount of the active ingredient, which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

It is a finding of the present invention that a profound analgesic effect may be observed even if the opioid analgesic drug is administered to the patient at a low dose which provides little or no analgesic effect when administered alone. Thus, the dosage of opioid analgesic drug administered in combination with the sodium channel blocker that inhibits Naᵥ1.7 is typically a low clinical dosage. This has significant advantages, particularly in terms of long-term treatment, since the well-known side effects of opioid analgesic drugs, such as addiction, constipation and respiratory depression will be drastically reduced or removed. As discussed above, this also makes the combinations of the invention particularly suitable for use in treating patients who are intolerant or contraindicated against opioid analgesic drugs.

A sub-clinical dosage of opioid analgesic drug is a dosage, which provides the same, or substantially the same, effect as a placebo. Thus, a sub-clinical dose typically provides an analgesic effect within in the range of ±20%, preferably ±10%, of that observed with placebo. The amount of drug representing sub-clinical dosage will vary from opioid analgesic drug to opioid analgesic drug, and a suitable dosage can be easily determined by a skilled physician

Similarly, a suitable dosage of the enkephalinase inhibitor will vary from compound to compound, and a dosage can be determined by a skilled physician.

Similarly, a suitable dosage of the selective Naᵥ1.7 inhibitor will vary from compound to compound, and a dosage can be determined by a skilled physician.

Dosage regimens may be adjusted to provide the optimum desired response. For example, a single dose may be administered, several divided doses may be administered over time or the dose may be proportionally reduced or increased as indicated by the exigencies of the therapeutic situation. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Administration may be in single or multiple doses. Multiple doses may be administered via the same or different routes and to the same or different locations. Alternatively, doses can be via a sustained release formulation, in which case less frequent administration is required. Dosage and frequency may vary depending on the half-life of the drugs in the patient and the duration of treatment desired. For example, the enkephalinase inhibitor/opioid analgesic drug and selective Naᵥ1.7 inhibitor of the invention may be administered about once per 4 hours, once per day about once per three days or, preferably at longer intervals orally, as an injection or via a transdermal patch.

### Administration of further therapeutic agents

The combination of (a) an enkephalinase inhibitor and optional opioid analgesic drug, and (b) a selective Naᵥ1.7 inhibitor of the invention may be administered in combination with one or more other therapeutic agents. For example, the other agent may be another analgesic or an anaesthetic, immunosuppressant or anti-inflammatory agent. In particular, the combination of (a) an enkephalinase inhibitor and optional opioid analgesic drug, and (b) a selective Naᵥ1.7 inhibitor of the invention may be combined with other therapeutic agents for the treatment of pain, e.g. other analgesics and or antibodies directed to other targets involved in pain. Other analgesics may include common painkillers such as paracetamol and aspirin and/or non-steroidal antiinflammatories (NSAIDs) such as ibuprofen, pregabalin/gabapentin for neuropathic pain, and other approved analgesic drugs. Antibodies directed to other targets involved in pain may include, in particular, antibodies to nerve growth factor (NGF) or tumor necrosis factor (TNF).

Combined administration of two or more agents may be achieved in a number of different ways. Both may be administered together in a single composition, or they may be administered in separate compositions as part of a combined therapy. For example, one may be administered before, after or concurrently with the other.

The following Examples illustrate the invention.

### EXAMPLES

### Example 1 - Analysis of the expression of dorsal root ganglia mRNA transcripts in Naᵥ1.7 null mutant mice

Deletion of 2 exons of the *Scn9a* gene encoding Naᵥ1.7 leads to loss of channel function in mouse sensory neurons, and is caused by the crossing of mice expressing Advillin Cre (a pan DRG Cre-recombinase mouse) with a mouse that has a floxed *Scn9a* gene (Nassar et al 2004). Deletion of *Scn9a* resulted in a dramatic alteration in gene expression in a large number of genes particularly those associated with pain pathways such as Runx-1. This suggests that Naᵥ1.7 has a role in transcriptional regulation in sensory neurons, and the complete analgesia found in human Naᵥ1.7 mutants could be a consequence of this dysregulation, as well as loss of channel activity.

The list of dysregulated genes was interrogated, and it was found that preproenkephalin levels were massively increased in Naᵥ1.7 null mutant sensory neurons (see Table 1). This raised the possibility that human pain-free Naᵥ1.7 null mutants had high levels of endogenous opioids that contribute to their analgesia.

**Table 1 - Some upregulated gene transcripts in knock-out mice**

| **Fold change in normal mice** | **Gene Symbol** | **Gene Identity** |
|---|---|---|
| -1.95 | *Timeless* | timeless homolog (Drosophila) |
| -1.95 | *S100a9* | S100 calcium binding protein A9 (calgranulin B) |
| -1.96 | *Crtacl* | cartilage acidic protein 1 |
| -1.96 | *Smr2* | submaxillary gland androgen regulated protein 2 |
| -2.04 | *Camp* | cathelicidin antimicrobial peptide |
| -2.05 | *Kcnvl* | potassium channel, subfamily V, member 1 |
| -2.11 | *Nox4* | NADPH oxidase 4 |
| -2.13 | *Lcn2* | lipocalin 2 |
| -2.2 | *Crtacl* | cartilage acidic protein 1 |
| -2.36 | *Blnk* | B cell linker |
| -2.68 | *Ptgerl* | prostaglandin E receptor 1 (subtype EP1) |
| -2.82 | *Tmem173* | transmembrane protein 173 |
| -**3.44** | ***Penk*** | **Preproenkephalin** |

### Generating transgenic mice

Transgenic mice were generated as described in Nassar et al. 2004 and Minett et al. 2012.

### RNA extraction

Mice were culled using CO₂ before dissecting the DRG and immediately placing into RNAlater. The DRG were then spun down and transferred into 1ml TRIzol. DRG were left on ice for 3 minutes then homogenised for 3 minutes. The samples were spun through a QIAshredder mini column at 13,000 rpm for 2 minutes. The column was then removed, and spun for another 3 minutes. The supernatant was measured and transferred into a phase lock gel tube before adding chloroform:isoamyl alcohol 24:1 and shaking vigorously for 15 seconds. The tubes were left to stand for 3 minute at room temperature then Spun at 14,000 rpm for 15 min at 4°C. The upper colourless aqueous layer was measured and transferred into a fresh 1.5 ml tube before adding an equal volume of 70% ethanol to the aqueous phase. The samples were then transferred to an RNeasy MinElute spin column placed in a 2 ml collection tube and spun at 14,000 rpm for 15 seconds. The flow-through was discarded and 500µl RPE buffer was added to the spin column and spun at 14,000 rpm for 15 seconds. The flow-through was discarded and 500µl 80% buffer was added to the spin column and spun at 14,000 rpm for 15 seconds. The flow-through was discarded and the RNeasy MinElute column was spun at 14,000 rpm for 5 minutes. The RNeasy MinElute spin column was placed in a fresh 1.5 ml collection tube and 10µl RNase-free water was added directly to the center of the spin column membrane before being spun at 14,000 rpm for 2 min to elute the RNA. The RNA concentration was measured using a Nanodrop and store at -20 degrees Celsius.

### Microarray analysis

1µg (100ng/gl) samples were prepared for microarray analysis using Affymetrix GeneChip Mouse Genome 430 2.0 array. Data was analysed using Affymetrix Transcriptome Analysis Console software.

### Example 2 - Testing whether endogenous opioids contribute to the analgesia of pain free Naᵥ1.7 mutants

The analgesia found in Naᵥ1.7 null mutant mice was measured with the Hargreaves apparatus, which is a measure of acute thermal pain (Minett et al. 2011, Minett et al. 2012). It was found that the non-selective opioid receptor antagonist naloxone could partially reverse the pain free state in Naᵥ1.7 null mutant mice, implicating opioid-mediated analgesia in Naᵥ1.7 null mutants (Fig. 1A).

A combination of a sodium channel inhibitor that blocks Naᵥ1.7 and an opioid analgesic drug was then tested in mouse models of acute pain. When Phlotoxin-1 , a selective tarantula peptide Naᵥ1.7 channel blocker (1µg equivalent to 0.3nMol), or the opioid buprenorphine (1µg equivalent to 2nMol), were administered alone as intraplantar injections, no analgesia was observed compared with control mice in the Hargreaves test. Surprisingly, when the two drugs were combined at the same dosage, profound analgesia developed within minutes that precisely matched the behaviour of mice with *Scn9a* deletion and no functional Naᵥ1.7 channel (Fig. 1B and 1C).

These data suggest that analgesia found in Naᵥ1.7 null mutant mice and humans is a result of both loss of channel activity and upregulation of the endogenous opioid system. It follows that combination therapy with an opioid analgesic drug combined with a Naᵥ1.7 blocker will be an effective treatment for acute, inflammatory and neuropathic pain that have been shown to be Naᵥ1.7 dependent (Minett et al. 2012).

To extend these observations a model of inflammatory pain was examined - the thermal hyperalgesia caused by Complete Freund's Adjuvant injections. A dramatic synergy in the analgesic effects of Phlotoxin-1 and buprenorphine, also lead to profound analgesia (Fig. 2).

### Behavioural assays

Thermal nociceptive thresholds were measured using the paw-withdrawal latency according to the method described by Hargreaves et al. (1988), with minor modifications (Minett et al. 2011). Briefly, mice were left to acclimatise within an IITC Hargreaves stand for at least 1 hour. Three withdrawal responses were measured per animal. The mice were then left to resettle for at least 30 minutes before the drug treatment.

### Complete Freund's Adjuvant

Baseline thermal thresholds were recorded for each animal before 20µl Complete Freund's Adjuvant (Sigma) was injected subcutaneously into the plantar aspect of a hind paw using a Hamilton syringe and 30G needle. Three days post Complete Freund's Adjuvant injections thermal threshold were measured before to drug treatment.

### Naloxone

Mice received intraperitoneal injections of either 2mg/kg naloxone or the equivalent volume of vehicle.

### Drug treatment

Mice received 20µl intraplantar injections of either;
1µg Phlotoxin-1,
1µg Buprenorphine,
combined 1µg Phlotoxin-1 and 1µg Buprenorphine
or vehicle.

### Example 3 - Measuring inhibition of Naᵥ1.7

The activity and/or selectivity of a particular compound as a Naᵥ1.7 inhibitor is determined as described in Farmer et al. 2012.

Specifically, HEK293 (ATCC) cells are transfected with Naᵥ1.7 or other sodium channel cDNA constructs in a vector co-expressing a fluorescent marker protein using lipofectamine 2000 or other transfection method and plated onto poly-d-lysine coated coverslips. All recordings are done 24-72 hours after transfection

Whole-cell voltage clamp recordings are performed using an Axopatch 200 B amplifier at room temperature using standard techniques. Extracellular solution comprised (mM) NaCl 145, KCl 4, CaCl₂ 1.8, MgCl₂ 1, HEPES 10, (pH 7.35 with NaOH); intracellular (mM) CsCl 150, EGTA 10, HEPES 10, (pH 7.35 with CsOH). Data was sampled at 50 kHz, filtered at 5 kHz and leak currents were subtracted using a P/4 protocol. Average series resistance was 5.1±0.3 MΩ and was compensated by 75-90%. Tau of inactivation was measured by fitting a standard exponential function to the decaying current and persistent current was determined by measuring the current 20 ms after the start of the voltage step and expressing it as a percentage of the transient current in the same step. To assess voltage dependence of activation current-voltage families were obtained and peak sodium currents measured at each voltage. Channel conductance was calculated using the formula G_{Na} = I/(V-Vrev) where G_{Na} is sodium conductance, I is peak current, V is the voltage step and Vrev is the measured reversal potential which was determined for each cell by fitting a straight line through the linear portion of the current-voltage relationship. Sodium conductances were normalized and fitted with a standard Boltzmann function: G_{Na}/G_{Na}max = 1/(1+ exp((V₅₀-V)/k)) where G_{Na}/G_{Na}max is the normalized conductance, V₅₀ is the voltage which produces half maximal conductance and k is the slope factor.

Potential sodium channel blockers are screened on expressed human Naᵥ1.7 sodium channels, as well as Naᵥ1.1, Naᵥ1.3 Naᵥ1.4, Naᵥ1.5 and Naᵥ1.6 and other channels to assess specificity. The compounds are bath applied at different concentrations to assess channel block.

### Example 4 - Thiorphan and Phlotoxin- 1

24 C57/B16 mice (12 males) were individually housed in Hargreaves test chambers (as described above in Example 2) and acclimatized prior to testing. Thermal nociceptive thresholds were measured using the paw-withdrawal latency to the Hargreaves test (see Example 2). A heat ramp of approximately 2°C.s⁻¹ was used.

Following determination of a baseline threshold, mice received a 10µl intraplantar injection of either saline, the Naᵥ1.7 antagonist Phlotoxin-1 (1µg)), the enkephalinase antagonist Thiorphan (20mg/kg) or a combination of Phlotoxin- 1 and Thiorphan (6 mice per group).

Thermal nociceptive thresholds were then measure 30 minutes post injection. The data were analysed by two-way analysis of variance followed by the Bonferroni post hoc test. The results are presented in Figure 3 as mean ± S.E.M. ** *P* < 0.01 and *** *P <* 0.001 (individual points).

### Example 5 - Measuring inhibition of enkephalinases

The activity of a particular compound as an enkephalinase inhibitor is determined as described in Florentin et al (1984) with modifications.

Specifically, fresh mouse kidney and spinal cord are homogenized in 10 vol of cold 0.05 M Tris-HCl buffer, pH 7.4, using a Polytron homogenizer. The homogenate is centrifuged for 5 min at 1000g. The pellet is discarded and the supernatant centrifuged at 60,000g for 60 min. The resulting pellet is resuspended in 50 mM Tris-HCl buffer, pH 7.4, and used as the enzyme source. Standard assays for enkephalinase activity using dansyl-D-Ala-Gly-Phe(pNO2)-Gly (DAGNPG) are carried out at 37°C in hemolysis tubes.

A 0.1-ml amount of 50 mM Tris-HCl buffer, pH 7.4, containing 50 mM DAGNPG is preincubated 15 min at 37°C. The reaction is initiated by addition of 50 ml of the enzyme preparation together with 0.5 mM Captopril. The tubes are incubated for 30 min in a water bath with constant shaking. The enzymatic reaction is stopped by boiling at 100°C for 5 min.

The samples are then diluted with 1.35 ml of Tris HCl buffer and centrifuged at 500g for 30 min. An aliquot of 1 ml of the supernatant is transferred to thermostated cells of a spectrofluorometer. Readings are performed at 562 nm with an excitation wavelength of 342 nm. A calibration curve is prepared by adding increasing concentrations of DNS-D-Ala-Gly and decreasing concentrations of the substrate in Tris-HCl buffer containing the denaturated enzymatic preparation. Active compounds are further evaluated using human enkephalinase preparations.

### Example 6 - Human studies of Naᵥ1.7 loss-of-function congenital insensitivity to pain

A 39 year old Naᵥ1.7 loss of function pain-free female (ML) was tested under baseline, saline, and naloxone (6mg) bolus and 0.1 mg/minute infusion treatment. The forearm was stimulated with radiant heat (CO₂ lasers).

### Detection thresholds

Warmth threshold reflects activation of unmyelinated C-fibres, while pinprick threshold is mediated by small-myelinated A-delta fibres (LaMotte et al 1978). Detection thresholds were assessed by slowly increasing the temperature of the stimulus (rate 1 deg/sec), after a neutral baseline of variable length (5-10 seconds). The participant was asked to press a button, to stop the stimulus as soon it was detected as warm or pinprick. At baseline, ML perceived only 1 of the 6 stimulus ramps. All the stimuli were detected in the subsequent saline and naloxone sessions.

Warmth detection thresholds were reduced by 7°C with naloxone relative to the saline conditions, in a range comparable with healthy volunteers. Pinprick detection thresholds were also reduced (1.7°C), with naloxone relative to the saline condition. Only with naloxone, ML reported the pinprick stimuli to be *"surprisingly uncomfortable"* (in 66% of the trials).

### Processing of tonic radiant heat

We delivered tonic stimuli, at the constant temperature of 42, 45, and 48°C for 20 sec. ML rated online fluctuations in perceived intensity on a visual analog scale. Identical stimuli were rated higher after administration of naloxone in comparison to both the baseline and saline conditions.

Furthermore, we measured the electroencephalogram (EEG) responses induced by tonic radiant heat stimulation: in other words, we looked at the effect of the nociceptive stimulation on brain rhythms.

With naloxone alpha and beta power was reduced reflecting processing of incoming nociceptive input (Ploner et al 2006). There was also an enhancement of both low and high gamma induced oscillations in the naloxone condition relative to baseline and saline. Enhancement of gamma oscillations over primary sensory cortices is associated with awareness of perceptual stimuli, in several modalities (Melloni et al 2007) including pain (Zhang et al 2012 and Gross et al 2007).

### Processing of phasic radiant heat

Laser pulses of the same energy (fluency 0.6 j/mm²) were given to the forearm. The energy used typically evokes a sensation of sharp pinprick pain in healthy volunteers. ML did not perceive any of the given laser pulses in the baseline and saline condition. However, naloxone enhanced the detection rate of laser pulses to 80%. ML felt 37.5% of the detected stimuli as pinprick, while the other sensations were described as a slow heat, or warmth. Ratings were low (mean 6.1, range 0.5-15) in a perceived intensity scale ranging from 0-100.

### Bodily awareness

We administered the McGill pain questionnaire three times. During baseline and saline, ML reported few tickles and tingles to both the hand and legs, possibly related to the posture. None of those was described as unpleasant. During the naloxone session, ML referred to unpleasant burning sensations on the stimulated arm, painful cramps in her injured left calf, and sore in the area were the needle was inserted.

### Tactile sensitivity

The test shows that the naloxone effects are specific for nociceptive stimulation. Tactile sensitivity was normal in ML, and remained unchanged in the three conditions. This underscores the specific association between Naᵥ1.7 and pain sensations rather than other non-noxious stimuli such as touch.

These experiments show that opioid analgesia contributes substantially to congenital insensitivity to pain in human *SCN9A* Naᵥ1.7 loss-of-function mutants, in a similar manner to that observed in Naᵥ1.7 null mutant mice.

### Example 7 - expression of Met-enkephalin peptides

Because of the high levels of proenkephalin precursor mRNA *Penk1* observed in Example 1, we examined the expression of Met-enkephalin peptides in the dorsal root ganglia and spinal cord of Naᵥ1.7 null mutant and normal mice. Using polyclonal antibodies to met-enkephalin we found that levels of the peptide were much increased in both cell bodies and the central terminals of Naᵥ1.7 null mutant mice.

In particular, mice were anesthetized by an intraperitoneal injection of pentobarbital and then transcardially perfused with an ice-cold solution of paraformaldehyde 4 % prepared in PBS (PAF). Once dissected, DRG and Spinal Cord were post-fixed for 3h in 4°C PAF and then transferred overnight into 10 % sucrose solution for cryoprotection. Tissues were then embedded in OCT mounting medium (Tissue-Tek), frozen in liquid nitrogen and stored at - 80°C. 10µm and 30µm cryostat sections, for respectively DRG and SC, were picked up on SuperFrost Plus slides. After PBS washes, DRG and SC slides were incubated overnight at 4°C with rabbit anti-met-enkephalin polyclonal antibody (1:500, Millipore). Secondary antibody conjugated with Alexa Fluor 488 (1:500, Invitrogen) was used for immunofluorescence. DRG immunostaining intensity was quantified with Image J software (NIH).

The results are depicted in Figure 4, which shows that deleting Naᵥ1.7 increases Met-enkephalin immunoreactivity in the DRG and spinal cord of transgenic mice.

### Example 8 - inhibition of Naᵥ1.7 using Phlotoxin-1.

The results in Figure 5 confirm that Phlotoxin-1 1 at a concentration of 0.25µM inhibits functional Naᵥ1.7 voltage gated channel activity in a Naᵥ1.7 transfected cell line. A HEK293 cell line transfected with human cDNA encoding Naᵥ1.7 demonstrates an inward current in response to depolarisation. Whole cell voltage clamp experiments were used to measure Naᵥ1.7 activity in these cells.

Application of Phlotoxin-1 to the cells completely blocks the voltage-gated sodium channel activity that results from Naᵥ1.7 expression.

### Example 9 - formalin pain tests

15 microliters of either saline or Phlotoxin-1 and buprenorphine was injected into the left hindpaw for a mouse (intraplanter) using a Hamilton syringe and 30G needle 30 minutes before 10 microlitres of 5% formalin was injected into the left hindpaw (intraplanter). Spontaneous nocifensive behavioural responses were recorded for 45 min post injection. The observer was blind to treatment. (Minett et al. 2011)

The results are depicted in Figure 6, and show that both first and second phase pain is lowered by the combination of Phlotoxin-1 and buprenorphine.

### Example 10 - Neuropathic pain

### Neuropathic Behavioural Phenotyping

Mechanical and thermal baseline thresholds were recorded using von Frey and Acetone tests (Minett et al. 2011). The various models of peripheral neuropathy were adapted for use on mice.

### Spinal nerve transection (of the 5^{th} lumbar vertebrae)

Animals were anaesthetised with isofluorene before a midline incision was made in the skin of the back at the L2-S2 levels and the left paraspinal muscles separated from the spinal processes, facet joints and transverse processes at the L4-S1 levels. A 2mm section was removed from the left L5 spinal nerve. The muscle and skin were closed in layers, using 3-0 sutures and surgical staples (Mabuchi et al., 2003; 2004). Thermal and mechanical thresholds were then monitored up to 28 days post-surgery (Minett *et al*., 2011).

### Cold allodynia

Behavioural response to cooling (approx. a drop of 10-15°C from skin temperature) by acetone test was performed as described by Bautista et al. (2006).

### Results

The results are depicted in Figure 7, from which it can be seen that Phlotoxin-1 and buprenorphine reverse neuropathic pain measured with cold allodynia and mechanical allodynia assays. In both cases, intraplantar treatment with Phlotoxin-1 (1 microgram) and Buprenorphine (1 microgram) restores the baseline situation, showing that neuropathic pain has been blocked.

### Example 11 - Osteoarthritis pain (21 days) is reversed by Phlotoxin-1 plus buprenorphine

To induce osteoarthritis, C57 BL/6 mice were anaesthetised and given an intra-articular injection of 0.5mg MIA (monoiodoacetate, Sigma) in 5µl 0.9% Saline into the knee.

Baseline von Frey testing was performed prior to injection and repeated at day 21 using the Chaplan 'Up-down' method (Minett *et al*, 2011).

At day 21, mice were given intraplantar injection of Phlotoxin-1 (1 microgram) + buprenorphine (1 microgram) or saline. von Frey testing was performed after 30 minutes. The tester was blind to the treatment.

As can be seen from Figure 8, treatment with Phlotoxin-1 plus buprenorphine restored the baseline situation, thus reversing osteoarthritis pain.

### Example 12 - Complete Freund's adjuvant (CFA) evoked inflammatory pain

Baseline thermal thresholds were recorded using the Hargreaves apparatus for each mouse. 20 microlites Complete Freund's adjuvant was injected into the left hindpaw of each mouse (intraplanter) using a Hamilton syringe and 30G needle. Sensitised behavioural responses were recorded using the Hargreaves apparatus, prior to injection of either saline, Phlotoxin-1, buprenorphine or Phlotoxin-1 and buprenorphine in combination. The observer was blind to treatment. (See Nassar et al 2004.)

The results are depicted in Figure 9, which shows that the combination of Phlotoxin- 1 and buprenorphine blocks CFA-evoked pain for hours after local intraplantar injection

### Example 13 - Intrathecal injection

Anesthetized mice were intrathecally injected with 7.5 microliters of either saline, Phlotoxin- 1 (0.025nMols , 0.1micogram), buprenorphine (1 microgram, 2nMols) or Phlotoxin-1 and buprenorphine in combination. Thermal thresholds were recorded using the Hargreaves apparatus for each animal. The observer was blind to treatment. (Eijkelkamp et al. 2013)

The results are depicted in Figure 10. These demonstrate that combination of Phlotoxin1 and buprenorphine synergistically blocks pain in this model.

### Example 14 - administration of naloxone to human patients

We evaluated the probability of detecting 9-ms radiant heat pulses in a Naᵥ1.7 null mutant human patient and in three age-matched, healthy human controls. Radiant heat was used to selectively stimulate intra-epidermal free-nerve endings, without touch. The results are depicted in Figure 11A.

The human Naᵥ1.7 null mutant could not detect any stimulus in the baseline and saline conditions. The probability of detecting the stimuli dramatically increased to 80% during the infusion of a bolus of 6 mg naloxone followed by an infusion at 0.1 mg/minute, almost reaching the detection levels of matched healthy controls.

Tonic radiant heat was administered over 25 seconds, while the participants rated online the intensity of the heat on a visual analogue scale. The results are depicted in Figure 11B. Naloxone strongly increased intensity ratings only in the Naᵥ1.7 null human, throughout the time course of the stimulation.

### References

The following references are discussed above
Bautista DM, Jordt SE, Nikai T, Tsuruda PR, Read AJ, Poblete J, Yamoah EN, Basbaum AI, Julius D. TRPA1 mediates the inflammatory actions of environmental irritants and proalgesic agents. Cell. 2006 Mar 24;124(6):1269-82.
Bosmans, F.Escoubas, P. Diochot, S. Cuypers, E. Mebs, D. Craik, D Hill, J. Nakajima, T. Lazdunski, M. Tytgat, J. Isolation and characterization of Phlotoxin 1, a novel peptide from spider venom acting selectively on voltage-gated Na+ channels Rencontres en Toxinology "toxins et douleur" edition: 13 location:Paris date: 1-2 December 2005
Cox JJ, Reimann F, Nicholas AK, Thornton G, Roberts E, Springell K et al. An SCN9A channelopathy causes congenital inability to experience pain. Nature 2006; 444: 894-898.
Eijkelkamp N, Linley JE, Baker MD, Minett MS, Cregg R, Werdehausen R, Rugiero F, Wood JN. Neurological perspectives on voltage-gated sodium channels Brain. 2012; Sep;135(Pt 9):2585-612.
Escoubas P Bosmans F., Cuypers E., Diochot S., Mebs D., Craik D., Hill J., Nakajima T., Lazdunski M, Tytgat j. Phlotoxin 1 a toxin from tarantula venom is a potent modulator of Nav1.7 sodium channels and a potential analgesic. poster presentation at 15th World Congress on Animal, Plant and Microbial Toxins, Glasgow, Scotland, 23-28 July 2006).
Farmer C, Cox JJ, Fletcher EV, Woods CG, Wood JN, Schorge S. Splice variants of Na(V)1.7 sodium channels have distinct β subunit-dependent biophysical properties. PLoS One. 2012;7(7):e41750. doi: 10.1371/journal.pone.0041750. Epub 2012 Jul 24.
Fertleman CR, Baker MD, Parker KA, Moffatt S, Elmslie FV, Abrahamsen B et al. SCN9A mutations in paroxysmal extreme pain disorder: allelic variants underlie distinct channel defects and phenotypes. Neuron 2006; 52: 767-774.
Florentin D, Sassi A, Roques BP (1984) A highly sensitive fluorimetric assay for "enkephalinase", a neutral metalloendopeptidase that releases tyrosine-glycine-glycine from enkephalins. Anal Biochem 141:62-69.
Gross J, Schnitzler A, Timmermann L, Ploner M. Gamma oscillations in human primary somatosensory cortex reflect pain perception. PLoS Biol. 2007;5:e133.
Hargreaves, K., Dubner, R., Brown, F., Flores, C. & Joris, J. (1988) A new and sensitive method for measuring thermal nociception in cutaneous hyperalgesia. Pain. 32 (1), 77-88.
LaMotte RH, Campbell JN. Comparison of responses of warm and nociceptive C-fiber afferents in monkey with human judgments of thermal pain. J Neurophysiol. 1978;41:509-28.
Lee JH, Park CK, Chen G., Han Q., Xie RG., Liu T., Jo RR. And Lee SY. (2014) A monoclonal antibody that targets a Nav1.7 channel voltate sensor for pain and itch relief Cell 2014.03.064 epub
Leipold E, Liebmann L, Korenke GC, Heinrich T, Giesselmann S, Baets J, Ebbinghaus M, Goral RO, Stodberg T, Hennings JC, Bergmann M, Altmüller J, Thiele H, Wetzel A, Nürnberg P, Timmerman V, De Jonghe P, Blum R, Schaible HG, Weis J, Heinemann SH, Hübner CA, Kurth I. A de novo gain-of-function mutation in SCN11A causes loss of pain perception. Nat Genet. 2013 Nov;45(11): 1399-404. doi: 10.1038/ng.2767.
Mabuchi, T., Matsumura, S., Okuda-Ashitaka, E., Kitano, T., Kojima, H., Nagano, T., Minami, T., and Ito, S. (2003). Attenuation of neuropathic pain by the nociceptin/orphanin FQ antagonist JTC-801 is mediated by inhibition of nitric oxide production. Eur J Neurosci 17, 1384-1392.
Mabuchi, T., Shintani, N., Matsumura, S., Okuda-Ashitaka, E., Hashimoto, H., Muratani, T., Minami, T., Baba, A., and Ito, S. (2004). Pituitary adenylate cyclase-activating polypeptide is required for the development of spinal sensitization and induction of neuropathic pain. J Neurosci 24, 7283-7291.
Melloni L, Molina C, Pena M, Torres D, Singer W, Rodriguez E. Synchronization of neural activity across cortical areas correlates with conscious perception. J Neurosci. 2007;27:2858-65.
Minett, M.S., Quick, K. & Wood, J.N. (2011) Behavioral Measures of Pain Thresholds. Johan Auwerx, Stephen D Brown, Monica Justice, David D Moore, Susan L Ackerman, & Joseph Nadeau (eds.). Hoboken, NJ, USA: Curr. Protoc. Mouse Biol. Online publication.
Minett MS, Nassar MA, Clark AK, Passmore G, Dickenson AH, Wang F et al. Distinct Nav1.7-dependent pain sensations require different sets of sensory and sympathetic neurons . Nat Commun 2012 Apr 24;3:791.
Nassar MA, Stirling LC, Forlani G, Baker MD, Matthews EA, Dickenson AH et al. Nociceptor-specific gene deletion reveals a major role for Nav1.7 (PN1) in acute and inflammatory pain. Proc Natl Acad Sci U S A 2004; 101: 12706-12711.
Ploner M, Gross J, Timmermann L, Pollok B, Schnitzler A. Pain suppresses spontaneous brain rhythms. Cereb Cortex. 2006;16:537-40.
Roques BP Fournie-Zaluski MC and Wurm M Inhibiting the breakdown of endogenous opioids and cannabinoids to alleviate pain. Nature Reviews drug discovery 2012, 292-310.
Tambaro S, Reali R, Volonterio A, Zanda M, Olimpieri F, Pinna GA, Lazzari P. NESS002ie: A new fluorinated thiol endopeptidase inhibitor with antinociceptive activity in an animal model of persistent pain. Pharmacol Biochem Behav. 2013 Jul 1;110C:137-144.
Taylor R Jr, Pergolizzi JV Jr, Porreca F, Raffa RB. Opioid antagonists for pain. Expert Opin Investig Drugs. 2013 Apr;22(4):517-25. doi: 10.1517/13543784.2013.778973.
van Ede F, Jensen O, Maris E. Tactile expectation modulates pre-stimulus beta-band oscillations in human sensorimotor cortex. Neuroimage. 2010;51:867-76.
Weiss J, Pyrski M, Jacobi E, Bufe B, Willnecker V, Schick B et al. Loss-of-function mutations in sodium channel Nav1.7 cause anosmia. Nature 2011; 472: 186-190.
Werdehausen R, Kremer D, Brandenburger T, Schlosser L, Jadasz J, Küry P, Bauer I, Aragón C, Eulenburg V, Hermanns H. Lidocaine metabolites inhibit glycine transporter 1: a novel mechanism for the analgesic action of systemic lidocaine? Anesthesiology. 2012 Jan;116(1):147-58.
Yang Y, Wang Y, Li S, Xu Z, Li H, Ma L et al. Mutations in SCN9A, encoding a sodium channel alpha subunit, in patients with primary erythermalgia. J Med Genet 2004; 41: 171-174.
Yang S, Xiao Y, Kang D, Liu J, Li Y, Undheim EA, Klint JK, Rong M, Lai R, King GF. Discovery of a selective NaV1.7 inhibitor from centipede venom with analgesic efficacy exceeding morphine in rodent pain models. Proc Natl Acad Sci U S A. 2013 Sep 30.
Zhang ZG, Hu L, Hung YS, Mouraux A, Iannetti GD. Gamma-band oscillations in the primary somatosensory cortex--a direct and obligatory correlate of subjective pain intensity. J Neurosci. 2012;32:7429-38.

## Claims

1. A pharmaceutical composition comprising:
(a) an enkephalinase inhibitor, and
(b) a selective Naᵥ1.7 inhibitor.

2. A pharmaceutical composition according to claim 1, which further comprises a an opioid analgesic drug.

3. A pharmaceutical composition according to claim 2, wherein the opioid analgesic drug is morphine, diamorphine, oxycodone, fentanyl, tramadol, buprenorphine, codeine, hydrocodone or hydromorphone.

4. A pharmaceutical composition according to any one of the preceding claims, wherein the enkephalinase inhibitor is thiorphan.

5. A pharmaceutical composition according to any one of the preceding claims, which is in unit dosage form.

6. A pharmaceutical composition according to any one of the preceding claims, wherein the selective Naᵥ1.7 inhibitor is an antibody that specifically binds Naᵥ1.7 and is selective for Naᵥ1.7 over other sodium channels.

7. A pharmaceutical composition as defined in any one of the preceding claims, for use in treating pain.

8. A pharmaceutical composition for use according to claim 7, wherein the pain is acute pain, inflammatory pain or neuropathic pain.

9. A pharmaceutical composition for use according to claim 7 or 8, wherein the pain is pain originating at peripheral sensory and/or sympathetic neurons.

10. An enkephalinase inhibitor as defined in claim 1 or 4 for use in treating pain in combination with a selective Naᵥ1.7 inhibitor as defined in claim 1 or 6, wherein the pain is as defined in any one of claims 7 to 9.

11. A selective Naᵥ1.7 inhibitor as defined in claim 1 or 6, for use in treating pain in combination with an enkephalinase inhibitor as defined in claim 1 or 4, wherein the pain is as defined in any one of claims 7 to 9.

## Patentansprüche

1. Pharmazeutische Zusammensetzung, umfassend:
(a) einen Enkephalinaseinhibitor und
(b) einen selektiven Naᵥ1.7-Inhibitor.

2. Pharmazeutische Zusammensetzung nach Anspruch 1, die ferner ein analgetisches Opioid-Arzneimittel umfasst.

3. Pharmazeutische Zusammensetzung nach Anspruch 2, wobei das analgetische Opioid-Arzneimittel Morphin, Diamorphin, Oxycodon, Fentanyl, Tramadol, Buprenorphin, Codein, Hydrocodon oder Hydromordphon ist.

4. Pharmazeutische Zusammensetzung nach einem der vorhergehenden Ansprüche, wobei der Enkephalinaseinhibitor Thiorphan ist.

5. Pharmazeutische Zusammensetzung nach einem der vorhergehenden Ansprüche, die in Einheitsdosierungsform ist.

6. Pharmazeutische Zusammensetzung nach einem der vorhergehenden Ansprüche, wobei der selektive Naᵥ1.7-Inhibitor ein Antikörper ist, der Naᵥ1.7 spezifisch bindet und für Naᵥ1.7 über andere Natriumkanäle selektiv ist.

7. Pharmazeutische Zusammensetzung nach einem der vorhergehenden Ansprüche zur Verwendung bei der Behandlung von Schmerzen.

8. Pharmazeutische Zusammensetzung zur Verwendung nach Anspruch 7, wobei die Schmerzen akute Schmerzen, Entzündungsschmerzen oder neuropathische Schmerzen sind.

9. Pharmazeutische Zusammensetzung zur Verwendung nach Anspruch 7 oder 8, wobei die Schmerzen Schmerzen sind, die an peripheren sensorischen und/oder sympathischen Neuronen ihren Ursprung haben.

10. Enkephalinaseinhibitor nach Anspruch 1 oder 4 zur Verwendung bei der Behandlung von Schmerzen in Kombination mit einem selektiven Naᵥ1.7-Inhibitor nach Anspruch 1 oder 6, wobei die Schmerzen wie in einem der Ansprüche 7 bis 9 definiert sind.

11. Selektiver Naᵥ1.7-Inhibitor nach Anspruch 1 oder 6 zur Verwendung bei der Behandlung von Schmerzen in Kombination mit einem Enkephalinaseinhibitor nach Anspruch 1 oder 4, wobei die Schmerzen wie in einem der Ansprüche 7 bis 9 definiert sind.

## Revendications

1. Composition pharmaceutique comprenant :
(a) un inhibiteur de l'enképhalinase, et
(b) un inhibiteur sélectif de Naᵥ1.7.

2. Composition pharmaceutique selon la revendication 1, qui comprend en outre un médicament analgésique opioïde.

3. Composition pharmaceutique selon la revendication 2, dans laquelle le médicament analgésique opioïde est la morphine, la diamorphine, l'oxycodone, le fentanyl, le tramadol, la buprénorphine, la codéine, l'hydrocodone ou l'hydromorphone.

4. Composition pharmaceutique selon l'une quelconque des revendications précédentes, dans laquelle l'inhibiteur de l'enképhalinase est le thiorphan.

5. Composition pharmaceutique selon l'une quelconque des revendications précédentes, qui est sous forme posologique unitaire.

6. Composition pharmaceutique selon l'une quelconque des revendications précédentes, dans laquelle l'inhibiteur sélectif de Naᵥ1.7 est un anticorps qui se lie spécifiquement à Naᵥ1.7 et est sélectif pour Naᵥ1.7 sur d'autres canaux sodiques.

7. Composition pharmaceutique telle que définie dans l'une quelconque des revendications précédentes, destinée à une utilisation dans le traitement de la douleur.

8. Composition pharmaceutique destinée à une utilisation selon la revendication 7, dans laquelle la douleur est une douleur aigüe, une douleur inflammatoire ou une douleur neuropathique.

9. Composition pharmaceutique destinée à une utilisation selon la revendication 7 ou 8, dans laquelle la douleur est une douleur générée au niveau des neurones sensoriels périphériques et/ou sympathiques.

10. Inhibiteur de l'enképhalinase tel que défini dans la revendication 1 ou 4 destiné à une utilisation dans le traitement de la douleur en combinaison avec un inhibiteur sélectif de Naᵥ1.7 tel que défini dans la revendication 1 ou 6, dans lequel la douleur est telle que définie dans l'une quelconque des revendications 7 à 9.

11. Inhibiteur sélectif de Naᵥ1.7 tel que défini dans la revendication 1 ou 6, destiné à une utilisation dans le traitement de la douleur en combinaison avec un inhibiteur de l'enképhalinase tel que défini dans la revendication 1 ou 4, dans lequel la douleur est telle que définie dans l'une quelconque des revendications 7 à 9.


### SEQUENCE LISTING

<110> UCL BUSINESS PLC
<120> COMBINATION OF ANALGESIC DRUGS
<130> N400981WO
<150> GB 1316468.6
   <151> 2013-09-16
<150> GB 1319595.3
   <151> 2013-11-06
<150> GB 1409272.0
   <151> 2014-05-23
<160> 2
<170> PatentIn version 3.5
<210> 1
   <211> 34
   <212> PRT
   <213> Theraphosidae
<400> 1
   Ala Cys Leu Gly Gln Trp Asp Ser Cys Asp Pro Lys Ala Ser Lys Cys
   1               5                   10                  15
   Cys Pro Asn Tyr Ala Cys Glu Trp Lys Tyr Pro Trp Cys Arg Tyr Lys
               20                  25                  30
   Leu Phe
<210> 2
   <211> 18
   <212> PRT
   <213> Artificial Sequence
<220>
   <223> Peptide fragment of Navl.7
<400> 2
   Leu Ser Leu Val Glu Leu Phe Leu Ala Asp Val Glu Gly Leu Ser Val
   1               5                   10                  15
   Leu Arg